## Europäisches Patentamt

(19) **European Patent Office**

**Office européen des brevets**

(11) Veröffentlichungsnummer: **0 067 794**
**A1**

(12) EUROPÄISCHE PATENTANMELDUNG

(21) Anmeldenummer: **82810267.3**

(22) Anmeldetag: **16.06.82**

(51) Int. Cl.³: **F 16 K 7/06**
**A 61 M 5/14**

(30) Priorität: **17.06.81 CH 4000/81**

(43) Veröffentlichungstag der Anmeldung:
**22.12.82 Patentblatt 82/51**

(84) Benannte Vertragsstaaten:
**AT BE CH DE FR GB IT LI LU NL SE**

(71) Anmelder: **Solco Basel AG**
**Gellertstrasse 18**
**CH-4052 Basel(CH)**

(72) Erfinder: **Laszczower, Max, Dr.**
**Gellertstrasse 74**
**CH-4052 Basel(CH)**

(74) Vertreter: **Eschmann, Heinz et al,**
**A. Braun, Braun, Héritier, Eschmann AG Patentanwälte**
**Holbeinstrasse 36-38**
**CH-4051 Basel(CH)**

(54) Vorrichtung zum Abklemmen von Schläuchen.

㉗ Eine aus zwei Klemmbügeln (1, 2) bestehende Schlauchklemme lässt sich durch Zusammenwirken einer Zunge (6) mit einer Zahnlippe (5) in ihre Anpressstellung bringen und verankern. Der abzuklemmende Schlauch lässt sich entweder durch zwei koaxiale Oeffnungen (10, 11) oder quer dazu zwischen den beiden Klemmbügeln (1, 2) anbringen. Demgemäss ist die Klemme im einen Falle sicher am Schlauch verankert und von diesem nur über ein freies Schlauchende abstreifbar, während sie sich im anderen Falle nach Belieben vom Umfang des Schlauches her frei anbringen und jederzeit lösen lässt.

Beide Anbringungsarten werden in der Praxis, insbesondere beim Absaugen und bei der Reinfusion von Blut bei Operationen, abwechslungsweise benötigt. Die Klemme ist einschliesslich sämtlicher Funktionsteile einteilig aus Kunststoff spritzbar.

FIG.1

## Vorrichtung zum Abklemmen von Schläuchen

Die vorliegende Erfindung betrifft eine Vorrichtung zum Abklemmen biegsamer, flüssigkeitsführender Schläuche zwecks Unterbindung bzw. Regulierung des Durchflusses, mit zwei zusammenwirkenden Klemmbügeln, zwischen welchen der Schlauch eingeklemmt wird, wobei die beiden Klemmbügel über eine Gelenkstelle verschwenkbar miteinander verbunden sind und an den freien Enden beider Klemmbügel Sicherungsorgane zur Verankerung der Klemmbügel in der Abklemmstellung angeordnet sind.

Flüssigkeitsführende Schläuche, z.B. solche, die zum Absaugen und zur Reinfusion von Blut bei Operationen dienen, werden mittels spezieller Vorrichtungen abgeklemmt, die eine teilweise oder auch vollständige Drosselung des Durchflusses gestatten.

In vielen Fällen ist hierbei mindestens ein Schlauchende offen zugänglich, während es andere Anwendungen gibt, in welchen der Schlauch an beiden Enden an Geräte bzw. Behälter angeschlossen ist und dies auch über längere Zeit bleibt.

Im letzteren Falle, in welchem kein offenes Schlauchende vorliegt, lässt sich eine Klemmvorrichtung nur vom Umfang des Schlauches her anbringen und ist auch jederzeit wieder lösbar; bleibt die Klemme jedoch über längere Zeit am Schlauch, so wäre es gerade hier wünschenswert, eine sicher am Schlauch verankerte Klemme zu haben, die sich zwar beliebig verstellen und auch in Richtung der Schlauchachse verschieben lässt, an sich aber nicht ohne Lösen eines Schlauchendes abgenommen werden kann. Dennoch sollte es aber möglich sein, diese Klemme für andere Anwendungsfälle von aussen her so am Schlauch anzubringen, dass sie jederzeit abgenommen werden kann, ohne dass hierzu eines der Schlauchenden gelöst werden müsste.

Diese von der Praxis geforderte Flexibilität der Anwendung weist keine der bekannten Schlauchklemmen auf. Es ist daher die Aufgabe der vorliegenden Erfindung, eine Vorrichtung zum Abklemmen biegsamer Schläuche vorzuschlagen, welche sowohl am Schlauch sicher verankert als auch leicht abnehmbar angebracht werden kann, wobei von Fall zu Fall entschieden werden kann, welche der beiden oben erwähnten Anbringungsarten vorteilhafterweise zur Anwendung kommen soll.

Diese Aufgabe wird erfindungsgemäss durch die im unabhängigen Patentanspruch 1 definierte Merkmalskombination gelöst.

Nachstehend wird anhand der beiliegenden Zeichnung ein Ausführungsbeispiel des Erfindungsgegenstandes beschrieben.

Fig. 1 ist eine Perspektivdarstellung einer bevorzugten Ausführungsform einer Vorrichtung zum Abklemmen eines biegsamen, flüssigkeitsführenden Schlauches,

Fig. 2 zeigt die erste Anbringungsart und

Fig. 3 die zweite Anbringungsart der Vorrichtung an einem abzuklemmenden Schlauch.

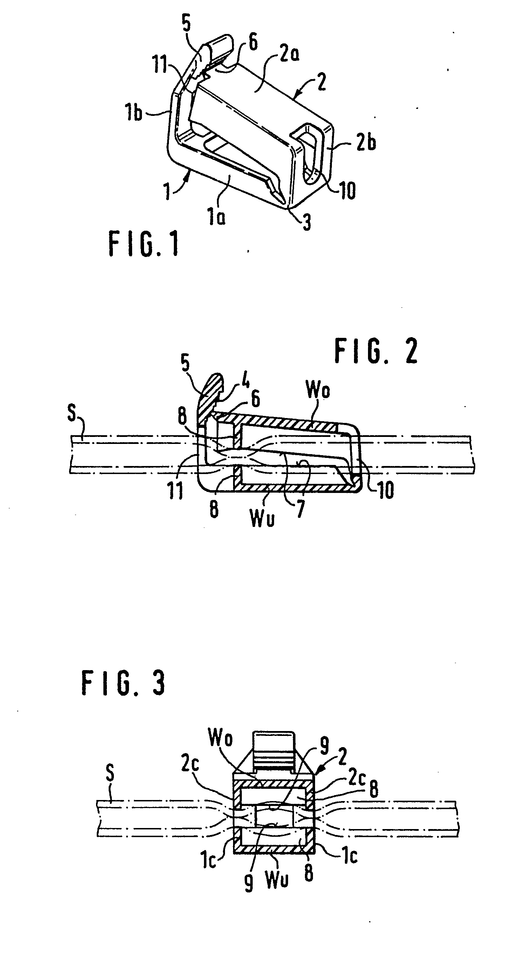

Gemäss Fig. 1 weist die Klemme zwei Klemmbügel 1 und 2 auf, welche durch ein Gelenk 3 miteinander verbunden sind. Beide Klemmbügel 1 und 2 sind bei einer bevorzugten Ausführungsform einteilig aus Kunststoff gefertigt, wobei das Gelenk 3 als Schwachstelle, d.h. als Stelle schwächeren Wandquerschnitts, ausgebildet ist.

Die beiden Klemmbügel 1 und 2 sind ähnlich, aber nicht identisch konstruiert und weisen beide - in ihrer Längsrichtung gesehen - einen praktisch L-förmigen Querschnitt auf. Demgemäss setzt sich der Klemmbügel 1 aus einem langen Schenkel 1a und einem kurzen Schenkel 1b, der Klemmbügel 2 aus einem langen Schenkel 2a und einem kurzen Schenkel 2b zusammen. An den kurzen Schenkel 1b des Klemmbügels 1 schliesst sich eine mit Zähnen 4 (Fig.2) versehene Zahnlippe 5 an, welche gegen die Vertikale leicht geneigt ist. Am freien Ende des langen Schenkels 2a ist dagegen eine spitz zulaufende Zunge 6 angeformt, welche in der Abklemmstellung in die Zahnung der Zahnlippe 5 eingreift.

Wie die Zeichnung ferner zeigt, sind die beiden Klemmbügel praktisch als offene Kastenprofile ausgebildet, d.h. von einer in Fig. 3 waagrechten oberen Wand Wo ragen jeweils 2 Seitenwangen 2c nach unten, während von der unteren Wand Wu zwei Seitenwangen 1c nach oben ragen. Die freien Kanten 7 dieser beiden Seitenwangenpaare dienen als Klemmkanten, wie sich aus der nachfolgenden Funktionsbeschreibung noch ergeben wird.

Wie ferner Fig. 2 zeigt, ist an jedem Klemmbügel ein nach unten bzw. oben ragender Steg 8 angeformt. Die beiden Stege 8 liegen in dem der Zahnlippe 5 benachbarten Teil der Klemme und ihre freien Kanten 9 haben ebenfalls die Funktion von Klemmkanten.

In jedem der beiden kleineren Schenkel 1b/2b ist ferner eine Oeffnung 10 bzw. 11 angebracht, die sich jeweils noch etwas über den angrenzenden längeren Schenkel 1a/2a erstreckt und deren freier Querschnitt mindestens dem vollen Querschnitt des abzuklemmenden Schlauches entsprechen soll.

Gemäss Fig. 2 lässt sich die beschriebene Klemme entsprechend der ersten Anbringungsart so verwenden, dass der Schlauch durch die beiden Oeffnungen 10 und 11 hindurchgesteckt und die Klemme durch Daumendruck auf die obere Wand Wo betätigt, d.h. verklemmt wird. Dabei drücken die Klemmkanten der beiden Stege 8 auf den Schlauch.

Man hält die Klemme dann praktischerweise zwischen Daumen und Zeigefinger und kann den gewünschten Durchflussquerschnitt durch den entsprechenden Fingerdruck regulieren, wobei die Zunge 6 dann in eine der vorgesehenen Zahnlücken der Zahnlippe 5 einrastet. Bei dieser Art der Anbringung ist die Klemme am Schlauch sicher verankert und kann sich auch nach dem Lösen des Abklemmdruckes nicht mehr von selbst lösen, zumal die Oeffnungen 10, 11 dem Schlauch einen gewissen Reibungswiderstand bieten. Die Klemme lässt sich somit zwar beliebig in Richtung der Schlauchachse verschieben, kann aber beispielsweise bei Operationen nicht abfallen, auch wenn das untere Schlauchende frei nach unten hängen sollte.

Die zweite Art der Anbringung zeigt Fig. 3. Hier ist die offene Klemme vom Umfang des Schlauches her über den Schlauch geschoben und dann wiederum zwischen Daumen und Zeigefinger betätigt, wobei hier die Klemmkanten der Seitenwangen 1c/2c den Schlauch je nach dem gewählten Druck abpressen. Aus dieser Stellung lässt sich die Klemme jederzeit beliebig abnehmen, ohne dass eines der eventuell angeschlossenen Schlauchenden gelöst werden müsste.

Die Praxis, insbesondere die bei Operationen durchzuführenden, zum Teil heiklen Manipulationen der Blutabsaugung und Reinfusion, zeigt, dass beide Arten der Anbringung wechselweise benötigt werden und dass die Vereinigung dieser beiden Systeme in einer einzigen Klemme nicht nur eine Reduktion der Anschaffungskosten, sondern auch mehr Sicherheit für den Patienten bringt.

Die Betätigung der beschriebenen Schlauchklemme ist äusserst unkompliziert und lässt dem Personal in jedem Falle eine Hand für andere Manipulationen frei. Das Lösen der einmal angebrachten Klemme erfolgt zweckmässigerweise dadurch, dass man die freie Vorderkante des kurzen Schenkels 1b mit der Spitze des Zeigefingers festhält und dann die Zahnlippe 5 mit dem auf der Wand Wo aufliegenden Daumen aufdrückt.

- 6 -

Die zur Erstellung einer solchen einteiligen Schlauchklemme erforderlichen verschleissfesten Kunststoffe sind dem Fachmann bekannt. Das Spritzteil weist sämtliche zur Funktion der Klemme erforderlichen Elemente auf, so dass nach dem Spritzvorgang keinerlei Montage oder sonstige Bearbeitung erforderlich ist.

In der Abklemmstellung nach Fig. 2 oder 3 wird die Zunge 6 durch eine elastische Rückstellkraft, die der Anpresskraft des Daumens beim Schliessvorgang entgegenwirkt, gegen die Zahnung 4 gepresst. Diese elastische Rückstellkraft wird vom Schlauch S ausgeübt, so dass die Anbringung eines entsprechenden Federelementes entfällt.

Patentansprüche

1. Vorrichtung zum Abklemmen biegsamer, flüssigkeitsführender Schläuche zwecks Unterbindung bzw. Regulierung des Durchflusses, mit zwei zusammenwirkenden Klemmbügeln, zwischen welchen der Schlauch eingeklemmt wird, wobei die beiden Klemmbügel über eine Gelenkstelle verschwenkbar miteinander verbunden sind und an den freien Enden beider Klemmbügel Sicherungsorgane zur Verankerung der Klemmbügel in der Abklemmstellung angeordnet sind, dadurch gekennzeichnet, dass die beiden Klemmbügel (1, 2) in ihrer Abklemmstellung zwei zueinander quer verlaufende Durchtritte für den Schlauch (S) freilassen und dementsprechend auch zwei zueinander querliegende Klemmkantenpaare (7, 9; 1c, 2c) vorgesehen sind, das Ganze derart, dass sich die Vorrichtung sowohl von einem Schlauchende her auf den Schlauch aufschieben als auch vom Schlauchumfang her um den Schlauch legen und in der entsprechenden Stellung sichern lässt.

2. Vorrichtung nach Anspruch 1, dadurch gekennzeichnet, dass die beiden Klemmbügel (1, 2) praktisch L-förmig ausgebildet sind und das eine Sicherungsorgan (5) eine Zahnung (4) aufweist, das andere Sicherungsorgan eine Zunge (6) ist, die dazu bestimmt ist, in der Abklemmstellung in die Zahnung (4) einzugreifen.

3. Vorrichtung nach Anspruch 2, dadurch gekennzeichnet, dass jeder Klemmbügel (1, 2) in seinem kürzeren Schenkel (1b/2b) eine Durchgangsöffnung (10, 11) aufweist und beide Durchgangsöffnungen (10, 11), welche in der Abklemmstellung praktisch miteinander fluchten, einen Schlauchdurchtritt bilden.

4. Vorrichtung nach einem der Ansprüche 1 und 2, dadurch gekennzeichnet, dass die längeren Schenkel (1a/2a) der beiden Klemmbügel (1, 2) als Kastenprofile ausgebildet sind, deren Seitenwände (1c/2c) Klemmkanten (7, 9) bilden.

5. Vorrichtung nach einem der Ansprüche 3 und 4, dadurch gekennzeichnet, dass jeder Klemmbügel (1, 2) mindestens einen die beiden Seitenwände (1c, 2c) verbindenden Steg (8) aufweist und die Lage der beiden Stege (8) so gewählt ist, dass dieselben als Klemmkanten für den Schlauch (S) dienen, wenn dieser durch die beiden Durchgangsöffnungen (10, 11) hindurchragt.

6. Vorrichtung nach einem der Ansprüche 1 bis 5, dadurch gekennzeichnet, dass dieselbe einteilig aus einem Kunststoff erstellt und das Gelenk (3) eine die beiden Klemmbügel (1, 2) verbindende Schwachstelle ist.

E

FIG.1

FIG. 2

FIG. 3

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-3 061 263   (BUTLER) | | F 16 K    7/06 <br> A 61 M    5/14 |
| A | FR-A-1 339 813   (POSSE) | | |
| A | US-A-4 097 020   (SOSSMAN) | | |

|  |
|---|
| RECHERCHIERTE SACHGEBIETE (Int. Cl. ³) |
| F 16 K    7/00 <br> A 61 M    5/00 |

Der vorliegende Recherchenbericht wurde für alle Patentansprüche erstellt.

| Recherchenort | Abschlußdatum der Recherche | Prüfer |
|---|---|---|
| DEN HAAG | 17-09-1982 | VERELST P.E.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1503 03.82